(19) Europäisches Patentamt
European Patent Office
Office européen des brevets

(11) EP 4 425 154 A1

(12) EUROPEAN PATENT APPLICATION
published in accordance with Art. 153(4) EPC

(43) Date of publication:
**04.09.2024 Bulletin 2024/36**

(21) Application number: **22887103.4**

(22) Date of filing: **27.10.2022**

(51) International Patent Classification (IPC):
***G01N 21/78*** *(2006.01)* ***A61K 49/00*** *(2006.01)*
***C07D 405/04*** *(2006.01)* ***C09B 57/02*** *(2006.01)*
***G01N 33/50*** *(2006.01)* ***G01N 33/92*** *(2006.01)*

(52) Cooperative Patent Classification (CPC):
**A61K 49/00; C07D 405/04; C09B 57/02; G01N 21/78; G01N 33/50; G01N 33/92**

(86) International application number:
**PCT/JP2022/040063**

(87) International publication number:
**WO 2023/074778 (04.05.2023 Gazette 2023/18)**

(84) Designated Contracting States:
**AL AT BE BG CH CY CZ DE DK EE ES FI FR GB GR HR HU IE IS IT LI LT LU LV MC ME MK MT NL NO PL PT RO RS SE SI SK SM TR**
Designated Extension States:
**BA**
Designated Validation States:
**KH MA MD TN**

(30) Priority: **27.10.2021 JP 2021175707**

(71) Applicant: **National University Corporation Gunma University**
**Maebashi-shi, Gunma 371-8510 (JP)**

(72) Inventors:
- **YOSHIHARA, Toshitada**
  **Maebashi-shi, Gunma 371-8510 (JP)**
- **SHIBUTA, Yuhi**
  **Maebashi-shi, Gunma 371-8510 (JP)**

(74) Representative: **Gill Jennings & Every LLP**
**The Broadgate Tower**
**20 Primrose Street**
**London EC2A 2ES (GB)**

(54) **FLUORESCENCE IMAGING REAGENT FOR LIPID DROPLETS IN TISSUES AND CELLS**

(57) An object of the present invention is to develop a fluorescent reagent capable of highly sensitive imaging of lipid droplets at levels ranging from cultured cells to individuals, which fluorescent reagent can be used in combination with a green fluorescent protein. The present invention provides a lipid droplet detection reagent including a compound represented by the following General Formula (I):

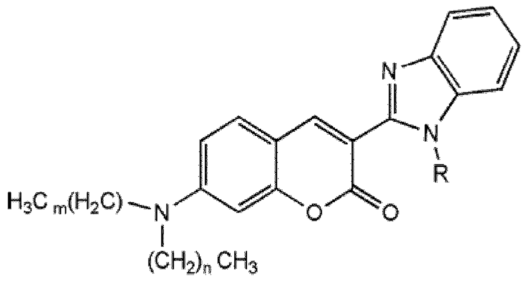

(I)

wherein
m represents an integer of 3 to 5;
n represents an integer of 3 to 5; and
R is $C_1$-$C_5$ alkyl, $C_6$-$C_{12}$ aryl, $C_7$-$C_{13}$ aralkyl, or hydrogen.

EP 4 425 154 A1

Fig. 4

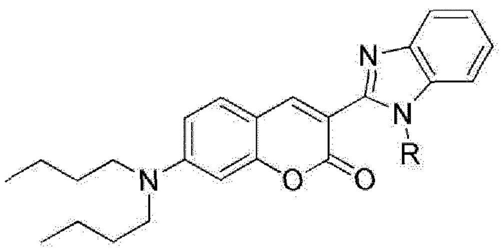

R : Me(DBC30)
Bu(DBC30-Bu)
Ph(DBC30-Ph)

## Description

TECHNICAL FIELD

The present invention relates to a fluorescence imaging reagent for lipid droplets in cells and tissues.

BACKGROUND ART

A lipid droplet (fat droplet) is a spherical organelle containing a neutral lipid such as triacylglycerol or cholesterol, surrounded with a monolayer phospholipid membrane. Although lipid droplets are found in a large amount mainly in adipocytes, they are ubiquitously present in any cells. Although the major role of lipid droplets has been thought to be storage of neutral lipid, a recent study showed that they are involved in regulation of intracellular lipid metabolism. There are also studies reporting about, for example, lipid droplets and autophagy. Thus, research on the mechanisms of the formation, growth, and degradation of lipid droplets is in progress. On the other hand, excessive accumulation of fat in a tissue (or in an individual) leads to dysfunction of the tissue, causing development of diabetes, arteriosclerosis, and the like. Further, in recent years, there is a sharp increase in the incidence of non-alcoholic steatohepatitis (NASH), which is a type of hepatitis. If NASH is left untreated, it may lead to liver cirrhosis or liver cancer. Therefore, elucidation of the mechanisms of the formation, growth, and degradation of lipid droplets in cells and tissues is important not only for cell biology, but also for diagnosis and treatment of these diseases. Thus, development of a molecular probe for highly sensitive real-time imaging of lipid droplets in living cells and tissues is required.

Fluorescence imaging is a method that simply enables imaging of living cells and tissues, and widely used in biological and medical studies. In the academic level, a number of fluorescent reagents for imaging of lipid droplets have been reported. However, only several kinds of reagents have been practically applied. Fig. 1 shows lipid droplet fluorescence imaging reagents that are commercially available at present. BODIPY 493/503 and Nile Red are used by many researchers. BODIPY 493/503 shows a green fluorescence at about 500 nm, and is highly selective for lipid droplets. However, they have problems such as poor photostability, low retention in lipid droplets, and leakage of excitation light due to a small Stokes shift (energy difference between the maximum absorption wavelength and the maximum fluorescence wavelength). Further, Nile Red has low selectivity for lipid droplets since it shows abundant distribution also in organelles other than lipid droplets. Moreover, since its absorption and fluorescence spectra largely vary depending on the surrounding microenvironment, multistaining with other fluorescent reagents is difficult. In order to solve the problems, LipiDye and the Lipi series (Lipi-Blue, Lipi-Green, Lipi-Red, and Lipi-Deep Red; the structural formula of Lipi-Deep Red has not been disclosed) were developed. Although these reagents are capable of selective imaging of intracellular lipid droplets, their capacity for imaging of lipid droplets in living tissues is unknown.

Regarding imaging of lipid droplets in living tissues, a nitrobenzene-substituted Nile Blue derivative (MNs-NB, Fig. 2) has been reported in Patent Document 1. In MNs-NB, photo-induced electron transfer reaction occurs between the nitrobenzene unit and Nile Blue in a polar solvent. On the other hand, in a low-polarity solvent, the photo-induced electron transfer reaction is less likely to occur, resulting in a red fluorescence. Although MNs-NB is a reagent capable of imaging of lipid droplets in tissues, it has problems such as a low fluorescence quantum yield (0.21; in chloroform) and a small Stokes shift.

The present inventors previously synthesized a reagent (PC6X, Fig. 3) for imaging of lipid droplets in tissues (adipose tissues, liver, kidney, and the like) of small animals under anesthesia (Patent Document 2). PC6X has a fluorescence quantum yield of not less than 0.8 in a solution, and has higher photostability than BODIPY 493/503 and Lipi-Green. Therefore, PC6X is capable of highly sensitive imaging of lipid droplets in cells and tissues over a long period of time. In addition, PC6X enables imaging of lipid droplets in the liver of a fatty-liver model mouse. However, since PC6X is a green fluorescent protein, it has a problem in the combined use with other green fluorescent proteins, which are widely used in the medical and biochemical fields.

PRIOR ART DOCUMENTS

[Patent Documents]

[Patent Document 1] JP 6241014 B
[Patent Document 2] WO 2020/189721

SUMMARY OF THE INVENTION

TECHNICAL PROBLEM

As described above, fluorescent reagents for lipid droplet imaging commercially available at present are limited to those for cultured cells. Moreover, MNs-NB, which is a commercially unavailable compound, has many problems to be solved before its practical application. In view of this, an object of the present invention is to develop a fluorescent reagent capable of highly sensitive imaging of lipid droplets at levels ranging from cultured cells to individuals, which fluorescent reagent can be used in combination with a green fluorescent protein. Such a fluorescent reagent may significantly contribute to the development of diagnostic agents and therapeutic agents for diseases caused by excessive lipid accumulation.

SOLUTION TO PROBLEM

As a result of intensive study to solve the above problems, the present inventors developed a blue-fluorescent reagent having a coumarin skeleton. Based on the discovery that use of this reagent enables selective fluorescence imaging of lipid droplets in cells and tissues, the present invention was completed.

Specifically, the present invention can be summarized as follows.

[1] A lipid droplet detection reagent comprising a compound represented by the following General Formula (I):

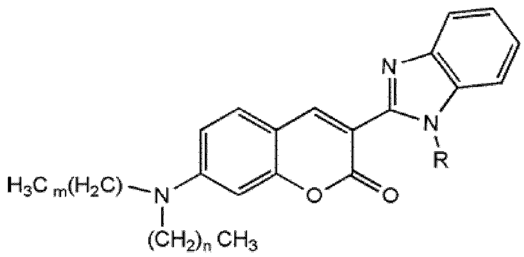

(I)

(wherein

m represents an integer of 3 to 5;
n represents an integer of 3 to 5; and
R is $C_1$-$C_5$ alkyl, $C_6$-$C_{12}$ aryl, $C_7$-$C_{13}$ aralkyl, or hydrogen).

[2] The detection reagent according to [1], wherein R is $C_1$-$C_4$ alkyl, or phenyl.
[3] The detection reagent according to [1] or [2], wherein m and n are 3.
[4] The detection reagent according to any of [1] to [3], for the detection of a lipid droplet in a biological sample.
[5] The detection reagent according to [4], wherein the biological sample is a cell or a tissue.
[6] The detection reagent according to any of [1] to [3], for the detection of a lipid droplet in a living individual.
[7] A lipid droplet detection method comprising the step of:
administering the detection reagent according to any of [1] to [6] to a biological sample or a living individual (other than a human, in one mode).
[8] Use of a compound represented by the General Formula (I) for the detection of a lipid droplet.
[9] Use of a compound represented by the General Formula (I) for the production of a lipid droplet detection reagent.
[10] A compound represented by the following General Formula (I)':

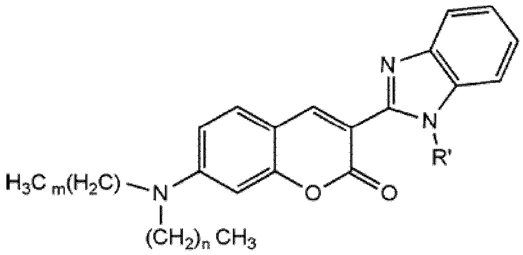

(I)'

(wherein

m represents an integer of 3 to 5;
n represents an integer of 3 to 5; and
R' is $C_1$-$C_5$ alkyl, $C_6$-$C_{12}$ aryl, or $C_7$-$C_{13}$ aralkyl).

[11] The compound according to [10], wherein R' is $C_1$-$C_4$ alkyl, or phenyl.
[12] The compound according to [10] or [11], wherein m and n are 3.

ADVANTAGEOUS EFFECTS OF THE INVENTION

According to the present invention, a blue-fluorescent reagent capable of selective fluorescence imaging of lipid droplets in cells or tissues can be provided.

BRIEF DESCRIPTION OF THE DRAWINGS

Fig. 1 shows the structural formulae of commercially available lipid droplet fluorescence imaging reagents.
Fig. 2 shows the structural formula of a Nile Blue derivative (MNs-NB).
Fig. 3 shows the structural formulae of the lipid droplet fluorescence imaging reagents described in Patent Document 2 (PC6X) or used in the Comparative Example (C6-OBu).
Fig. 4 shows the structural formula of the lipid droplet fluorescence imaging reagents described in the Examples in the present invention.
Fig. 5 shows the absorption and fluorescence spectra of compounds of the present invention (the DBC30 series).
Fig. 6 shows fluorescence imaging images obtained by adding the DBC30 series or a commercially available lipid droplet imaging reagent to HeLa cells (drawing-substituting photographs).
Fig. 7 shows the result of evaluation of the photostability of DBC30, C6-OBu, and commercially available lipid droplet imaging reagents in cells.
Fig. 8 shows a co-staining fluorescence imaging image obtained with DBC30 and a commercially available lipid droplet imaging reagent (BODIPY 493/503) (drawing-substituting photographs).
Fig. 9 shows a co-staining fluorescence imaging image obtained with DBC30-Bu and a commercially available lipid droplet imaging reagent (BODIPY 493/503) (drawing-substituting photographs).
Fig. 10 shows a co-staining fluorescence imaging image obtained with DBC30-Ph and a commercially available lipid droplet imaging reagent (BODIPY 493/503) (drawing-substituting photographs).
Fig. 11 shows a multicolor imaging image obtained with DBC30 and commercially available lipid droplet imaging reagents (Mito Tracker Green and Lyso Tracker Red) (drawing-substituting photographs).
Fig. 12 shows fluorescence intensity imaging images (Intensity) and fluorescence lifetime imaging images (FLIM) of adipose tissues and lipid droplets in a healthy mouse and a liver fat model mouse to which DBC30 was administered (drawing-substituting photographs).
Fig. 13 shows fluorescence imaging images of a subcutaneous adipose tissue, an abdominal adipose tissue, skeletal muscle, and kidney of a mouse to which DBC30 was administered (drawing-substituting photographs).

DESCRIPTION OF THE EMBODIMENTS

The present invention is described below.

<Lipid Droplet Detection Reagent>

One aspect of the present invention relates to a lipid droplet detection reagent (which may be hereinafter referred to as "lipid droplet detection reagent of the present invention") containing a compound represented by the following General Formula (I). The lipid droplet herein means a spherical droplet containing lipid, which droplet is contained, for example, in a cell.

The compound represented by General Formula (I) is a compound having the following structure.

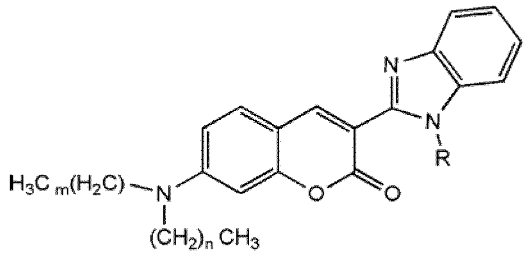

(I)

In General Formula (I), m represents an integer of 3 to 5. m is preferably 3 from the viewpoint of photophysical properties, the synthesis, and the like.

In General Formula (I), n represents an integer of 3 to 5. n is preferably 3 from the viewpoint of photophysical properties, the synthesis, and the like.

In General Formula (I), R is $C_1$-$C_5$ alkyl, $C_6$-$C_{12}$ aryl, $C_7$-$C_{13}$ aralkyl, or hydrogen.

The $C_1$-$C_5$ alkyl may be linear alkyl, branched alkyl, or cycloalkyl, such as methyl, ethyl, *n*-propyl, isopropyl, *n*-butyl, *s*-butyl, *t*-butyl, or pentyl.

The $C_6$-$C_{12}$ aryl may be substituted or unsubstituted aryl, such as phenyl, tolyl, xylyl, or naphthyl.

The $C_7$-$C_{13}$ aralkyl may be substituted or unsubstituted aralkyl, such as benzyl or phenethyl.

Examples of the substituents include, but are not limited to, $C_1$-$C_4$ alkyl, alkoxy, and halogens.

R is preferably $C_1$-$C_4$ alkyl, or phenyl from the viewpoint of photophysical properties, the synthesis, and the like.

Specific examples of the compound represented by General Formula (I) include the compounds listed below. However, the present invention is not limited thereto.

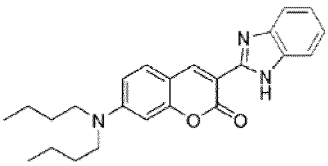

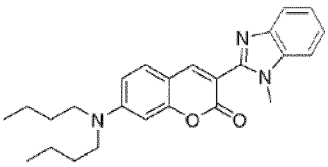

DBC30

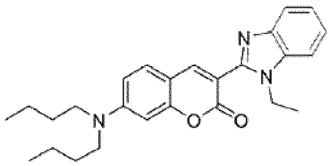

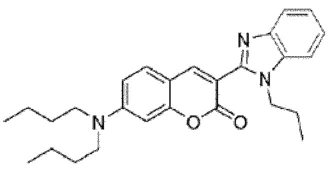

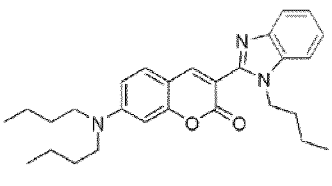

DBC30-Bu

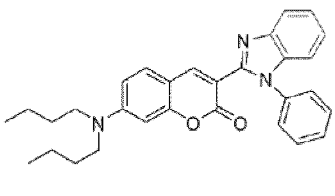

DBC30-Ph

Photophysical properties such as the maximum absorption/fluorescence wavelength, the fluorescence quantum yield ($\Phi_f$), and the fluorescence lifetime (iε) of the fluorescence of the compound represented by General Formula (I) can be measured by known measurement methods. For example, the maximum absorption/fluorescence wavelength and the fluorescence quantum yield can be measured using a luminescent quantum yield measurement apparatus or the like, for a sample in which the compound represented by General Formula (I) is dissolved in a solvent or the like. The fluorescence lifetime (iε) can be measured using a fluorescence lifetime measurement apparatus, for the compound in each solvent.

The fluorescence quantum yield ($\Phi_f$) is not limited, and may be changed in accordance with the structure of the compound, the type of the solvent, and the like. It is, for example, not less than 0.5, not less than 0.7, not less than

0.8, or not less than 0.9.

The fluorescence lifetime (if) is not limited, and may be changed in accordance with the structure of the compound, the type of the solvent, and the like. It is, for example, not less than 2.0 ns (nanoseconds), not less than 2.2 ns, not less than 2.4 ns, or not less than 2.5 ns.

The maximum excitation wavelength of the compound represented by General Formula (I) in a solvent is not limited, and may be changed in accordance with the structure of the compound, the type of the solvent, and the like. It is, for example, 390 nm to 460 nm. The maximum fluorescence wavelength in a solvent may also be appropriately set, and may be, for example, 460 nm to 500 nm.

«Method of Producing Compound»

The compound represented by General Formula (I) may be produced based on description in the later-described Examples or a known organic synthesis method.

<<Reagent>>

The lipid droplet detection reagent of the present invention comprises a compound having the structure described above. With this structure, the reagent can have excellent photophysical properties of fluorescence (regarding the blue fluorescence, fluorescence quantum yield, fluorescence lifetime, Stokes shift, and the like). In particular, since the reagent has the excellent photophysical properties described above in various solvents, it is useful as a lipid droplet detection reagent not only for cells, but also for living individuals. Further, with the above-described structure, the reagent can have excellent lipid droplet selectivity and intracellular retention property. Thus, the reagent can be used as a highly specific lipid droplet detection reagent.

The lipid droplet detection reagent of the present invention may be composed only of the compound represented by General Formula (I), or, as long as the effect of the present invention is not inhibited, the reagent may also contain a solvent, an additive, and a compound to be used as a lipid droplet detection reagent other than the compound used in the present invention.

<Lipid Droplet Detection Method>

One aspect of the present invention relates to a lipid droplet detection method comprising the step of administering the lipid droplet detection reagent of the present invention to a biological sample or a living individual (other than a human, in one mode) (which method may be hereinafter referred to as "lipid droplet detection method of the present invention").

Another aspect of the present invention relates to a lipid droplet detection method in which a solution containing: the lipid droplet detection reagent of the present invention; and a solubilizer; is administered to a biological sample or a living individual (other than a human, in one mode). The compound represented by General Formula (I) used as the lipid droplet detection reagent exhibits water insolubility in some cases. In such cases, the compound represented by General Formula (I) may be dissolved in an organic solvent in which the compound represented by General Formula (I) is soluble, and the resulting solution may be mixed with an aqueous solution containing a solubilizer, to prepare a solution. The prepared solution may then be administered to a biological sample or a living individual (other than a human, in one mode). The solubilizer is not limited as long as it is capable of giving water solubility to the compound represented by General Formula (I), and as long as the solubilizer has biocompatibility. Preferred examples of the solubilizer include biocompatible proteins such as albumin, gelatin, and casein. A single type of solubilizer, or a mixture of two or more types of solubilizers may be used. The solubilizer may be used at, for example, 1 to 30% by mass, preferably 5 to 20% by mass, more preferably 7.5 to 10% by mass in an aqueous solution. Adjustment may be appropriately carried out, but the compound may be used at, for example, 0.01 to 50 mM, preferably 0.1 to 5 mM, more preferably 0.5 to 1 mM in an organic solvent, or in a solution prepared by mixing an organic solvent in which the compound represented by General Formula (I) is soluble, with an aqueous solution containing a solubilizer.

The lipid droplet detection method of the present invention may further comprise the step of detecting the lipid droplet detection reagent of the present invention. The detection of the lipid droplet detection reagent may be carried out based on a known detection method for a fluorescent reagent.

The lipid droplet detection reagent of the present invention may be used, for example, as a detection reagent for detecting lipid droplets in a biological sample. The biological sample is not limited, and may be, for example, a cell or an isolated tissue. The lipid droplet detection reagent of the present invention may also be applied to, and detected in, a living body, and may be used as a detection reagent for detecting lipid droplets in a cell, tissue, or the like in a living individual.

The lipid droplet detection reagent of the present invention is capable of specifically detecting lipid droplets

present in a cell. Thus, the reagent is useful as a detection reagent for lipid droplets in a cell.

The detection of lipid droplets present in a cell may be carried out, for example, as follows.

The lipid droplet detection reagent of the present invention is added to a cell containing, or expected to contain, a lipid droplet.

Thereafter, a fluorescence signal of the lipid droplet detection reagent of the present invention is monitored using a fluorescence microscope or the like, to detect the lipid droplet contained in the cell.

The amount of the lipid droplet detection reagent of the present invention added to the cell may be appropriately changed in accordance with the cell used, the ratio of lipid droplets, and the like. The reagent may be added to the cell to a final concentration of, for example, 0.01 to 100 μM, preferably 0.1 to 10 μM.

In cases where the lipid droplet detection reagent of the present invention is added to the cell after dissolving the reagent in a solvent, examples of the solvent that may be used include, but are not limited to, organic solvents such as n-hexane, dibutyl ether, ethyl acetate, acetonitrile, and dimethyl sulfoxide.

The cell to which the lipid droplet detection reagent of the present invention is added is not limited as long as it is a cell containing, or expected to contain, a lipid droplet. Examples of the cell include 3T3-L1 cells and isolated adipocytes. Alternatively, the cell used may be a cell prepared by artificially forming lipid droplets in a cell free of lipid droplets or in a cell containing only a small amount of lipid droplets. Examples of the cell free of lipid droplets or the cell containing only a small amount of lipid droplets include HeLa cells, UEET-12 cells, and NIH3T3 cells. Examples of the method of forming lipid droplets include a method in which lipid droplets are induced by a method such as addition of oleic acid to a cell.

The lipid droplet detection reagent of the present invention is also capable of specifically detecting lipid droplets in a tissue, and lipid droplets and adipose tissues in a living individual (living organism individual). Thus, the reagent is useful as a detection reagent for lipid droplets in a tissue, and lipid droplets and adipose tissues in a living body.

The detection of lipid droplets present in a tissue may be carried out, for example, as follows.

The lipid droplet detection reagent of the present invention is added to a tissue containing, or expected to contain, a lipid droplet.

Thereafter, a fluorescence signal of the lipid droplet detection reagent of the present invention is monitored using a fluorescence microscope or the like, to detect the lipid droplet contained in the tissue.

The amount of the lipid droplet detection reagent of the present invention added to the tissue may be appropriately changed in accordance with the tissue used, the ratio of lipid droplets, and the like. The reagent may be added to the tissue to a final concentration of, for example, 0.01 to 100 μM, preferably 0.1 to 10 μM.

In cases where the lipid droplet detection reagent of the present invention is added to the tissue after dissolving the reagent in a solvent, examples of the solvent that may be used include, but are not limited to, organic solvents such as n-hexane, dibutyl ether, ethyl acetate, acetonitrile, and dimethyl sulfoxide. Further, the reagent may be administered in combination with a biocompatible liquid. Further, as described above, an organic solvent containing the lipid droplet detection reagent of the present invention may be mixed with an aqueous solution containing a solubilizer to prepare a solution, and the prepared solution may be added to the tissue.

Examples of the tissue subjected to the detection by the lipid droplet detection reagent of the present invention include, but are not limited to, subcutaneous fat, visceral fat, and ectopic fat (such as fat accumulated in an organ, for example, muscle, liver, heart, pancreas, or kidney).

The detection of lipid droplets present in a living individual may be carried out, for example, as follows.

The lipid droplet detection reagent of the present invention is administered to the living individual.

Thereafter, a fluorescence signal of the lipid droplet detection reagent of the present invention is monitored using a bioimaging method using a confocal microscope or the like, to detect adipose tissues in the living body in the living state without fixation of the living individual.

Examples of the dosage form of the lipid droplet detection reagent of the present invention include intravenous administration, subcutaneous administration, and intramuscular administration.

Although the dose of the lipid droplet detection reagent of the present invention may vary depending on the subject to which the reagent is to be administered, the dosage form, and the like, the reagent may be administered at a dose within the range of, for example, 0.01 to 1.0 μmol/kg body weight, preferably 0.1 to 0.5 μmol/kg body weight.

In cases where the lipid droplet detection reagent of the present invention is administered to the living individual after dissolving the reagent in a solvent, examples of the solvent that may be used include, but are not limited to, organic solvents such as n-hexane, dibutyl ether, ethyl acetate, acetonitrile, and dimethyl sulfoxide. Further, the reagent may be administered in combination with a biocompatible liquid. Further, as described above, an organic solvent containing the lipid droplet detection reagent of the present invention may be mixed with an aqueous solution containing a solubilizer, to prepare a solution, and the prepared solution may be added to the living individual.

Examples of the organism individual to which the reagent is to be administered include, but are not limited to, vertebrates including mammals (such as mice, humans, pigs, dogs, rabbits, and humans), and invertebrates.

<Compound of Present Invention>

The compound represented by the following General Formula (I)' is a novel compound synthesized by the present invention. Thus, one aspect of the present invention relates to a compound represented by the following General Formula (I)' (which may be hereinafter referred to as "compound of the present invention ").

The compound represented by General Formula (I)' is a compound having the following structure.

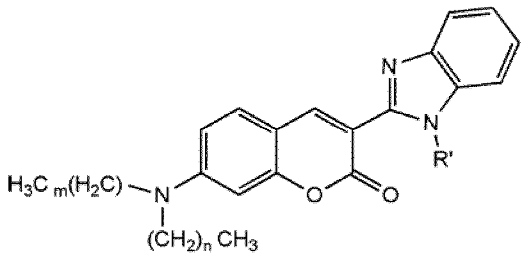

(I)'

In General Formula (I)', m represents an integer of 3 to 5. m is preferably 3 from the viewpoint of photophysical properties, the synthesis, and the like.

In General Formula (I)', n represents an integer of 3 to 5. n is preferably 3 from the viewpoint of photophysical properties, the synthesis, and the like.

In General Formula (I)', R' is $C_1$-$C_5$ alkyl, $C_6$-$C_{12}$ aryl, or $C_7$-$C_{13}$ aralkyl.

Specific examples of the alkyl, aryl, and aralkyl in R' are the same as those described in the <Lipid Droplet Detection Reagent> above.

R' is preferably $C_1$-$C_4$ alkyl, or phenyl from the viewpoint of photophysical properties, the synthesis, and the like.

EXAMPLES

The present invention is described below concretely by way of Examples. However, these are exemplifications of the present invention, and the scope of the present invention is not limited to these.

<Synthesis Examples>

The compounds DBC30, DBC30-Bu, and DBC30-Ph were synthesized as follows.

<<DBC30>>

7-Dibutylamino-2-oxo-2H-1-benzopyran-3-carboaldehyde (150 mg, 0.5 mmol) and *N*-methyl-1,2-benzenediamine (61 mg, 0.5 mmol) were added to 2-ethoxyethanol (15 mL), and the resulting mixture was stirred at 120°C for 24 hours. The solvent was evaporated under reduced pressure, and the resulting crude product was purified (silica gel column; developing solvent, n-hexane: ethyl acetate (1:1, v/v)) using a flash automatic purification apparatus (Isolera Spektra, Biotage), to obtain DBC30 (yield: 83 mg, 41%).

$^1$H NMR (400 MHz, $CDCl_3$, TMS): δ8.18(1H, s), 7.73-7.79(1H, d), 7.26-7.42(3H, m), 6.56-6.63(1H, d), 6.50(1H, s), 3.80(3H, s), 3.31-3.41(4H, t), 1.57-1.67(3H, m), 1.32-1.44(4H, m), 0.94-1.02(6H, t)
ESI-MS (m/z) of DBC30: calcd for $C_{25}H_{29}N_3O_2[M+H]^+$: 403.23, found: 404.3

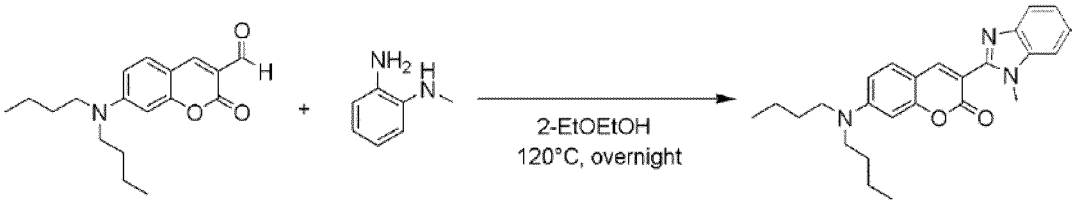

<<DBC30-Bu>>

7-Dibutylamino-2-oxo-2H-1-benzopyran-3-carboaldehyde (151 mg, 0.5 mmol) and *N*-butyl-1,2-benzenediamine (93 mg, 0.5 mmol) were added to 2-ethoxyethanol (15 mL), and the resulting mixture was stirred at 120°C for 24 hours. The solvent was evaporated under reduced pressure, and the resulting crude product was purified (silica gel column; developing solvent, n-hexane: ethyl acetate (1:1, v/v)) using a flash automatic purification apparatus (Isolera Spektra, Biotage), to obtain DBC30-Bu (yield: 47 mg, 20%).

$^1$H NMR (400 MHz, $CDCl_3$, TMS): δ8.13(1H, s), 7.73-7.80(1H, d), 7.38-7.45 (1H, m), 7.31-7.38 (1H, m), 6.55-6.63 (1H, d), 6.50 (1H, s), 4.20-4.30 (2H, t), 3.28-3.43 (4H, t), 1.72-1.86 (2H, m), 1.56-1.66 (4H, m), 1.32-1.45 (4H, m), 1.16-1.30 (2H, m), 0.94-1.02 (6H, t), 0.80-0.89 (3H, t)

ESI-MS (m/z) of DBC30-Bu: calcd for $C_{28}H_{35}N_3O_2[M+H]^+$: 445.27, found: 446.4

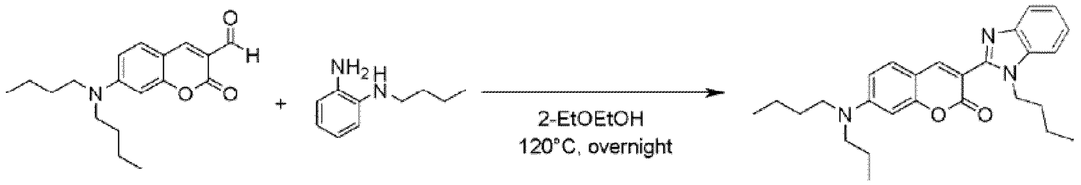


<<DBC30-Ph>>

7-Dibutylamino-2-oxo-2H-1-benzopyran-3-carboaldehyde (150 mg, 0.5 mmol) and *N*-phenyl-1,2-benzenediamine (270 mg, 1.6 mmol) were added to 2-ethoxyethanol (15 mL), and the resulting mixture was stirred at 120°C for 24 hours. The solvent was evaporated under reduced pressure, and the resulting crude product was purified (silica gel column; developing solvent, n-hexane: ethyl acetate (1:1, v/v)) using a flash automatic purification apparatus (Isolera Spektra, Biotage), to obtain DBC30-Ph (yield: 83 mg, 41%).

$^1$H NMR (400 MHz, $CDCl_3$, TMS): δ8.13 (1H, s), 7.76-7.93 (1H, d), 7.27-7.50 (6H, m), 6.48-6.61 (1H, d), 6.36 (1H, s), 3.20-3.42 (4H, t), 1.49-1.65 (4H, m), 1.27-1.45 (4H, m), 0.89-1.02 (6H, t)

ESI-MS (m/z) of DBC30-Ph: calcd for $C_{30}H_{31}N_3O_2[M+H]^+$: 465.24, found: 466.3

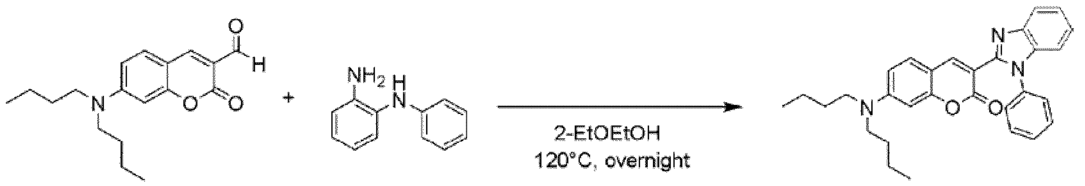


<Measurement Method>

(Measurement of Maximum Absorption Wavelength, Maximum Fluorescence Wavelength, and Fluorescence Quantum Yield)

Using a luminescent quantum yield measurement apparatus (C9920-01, manufactured by Hamamatsu Photonics K. K.), the maximum absorption wavelength ($\lambda_{abs}$/nm), the maximum fluorescence wavelength ($\lambda_{flu}$/nm), and the fluorescence quantum yield ($\Phi_f$) of the compound in each solvent were measured.

The absorption spectrum was measured using an ultraviolet and visible spectrophotometer (Ubest-550, manufactured by JASCO Corporation), and the fluorescence emission spectrum was measured using a fluorescence spectrophotometer (F-7000, manufactured by Hitachi, Ltd.)

(Measurement of Fluorescence Lifetime)

The fluorescence lifetime (iε) of the compound in each solvent was measured using a compact fluorescence lifetime measurement apparatus (Quntaurus-Tau, manufactured by Hamamatsu Photonics K. K.).

The fluorescence yield, that is, the fluorescence quantum yield ($\Phi_f$), represents the ratio of photons emitted as

fluorescence out of the photons absorbed in a substance. Thus, the higher the fluorescence yield, the higher the luminescence efficiency and the higher the luminescence intensity. The value of the fluorescence lifetime (iε) is unique to the molecule.

(Fluorescence Intensity Imaging and Fluorescence Lifetime Imaging)

Fluorescence intensity imaging and fluorescence lifetime imaging were carried out using a confocal laser microscope (IX-73; manufactured by Olympus Corporation) equipped with a confocal scanner (DCS-120; Becker & Hickl).

<Example 1>

The compounds of the present invention synthesized in the above Synthesis Examples (Fig. 4; DBC30, DBC30-Bu, and DBC30-Ph) contain an 8-dibutylaminocoumarine skeleton. Fig. 5 shows the absorption and fluorescence spectra of DBC30, DBC30-Bu, and DBC30-Ph in dibutyl ether or acetonitrile. Table 1 shows the photophysical parameters. The maximum absorption wavelength was found at 401 to 412 nm, and the maximum fluorescence wavelength was found at 467 to 484 nm, which corresponds to blue fluorescence. Each maximum wavelength exhibited a shift toward a longer wavelength as the polarity of the solvent increased. The fluorescence quantum yield was not less than 0.9 in any of the solvents.

Table 1 Photophysical properties of DBC30, DBC30-Bu, and DBC30-Ph

| probe | $\lambda_{abs}^{max}$ / nm | | $\lambda_{flu}^{max}$ / nm | | $\tau_f$ / ns | | $\phi_f$ | |
|---|---|---|---|---|---|---|---|---|
| | $Bu_2O$ | MeCN | $Bu_2O$ | MeCN | $Bu_2O$ | MeCN | $Bu_2O$ | MeCN |
| DBC30 | 404 | 410 | 468 | 484 | 2.29 | 2.55 | 0.93 | 0.90 |
| DBC30-Bu | 401 | 407 | 470 | 483 | 2.42 | 2.57 | 0.92 | 0.90 |
| DBC30-ph | 399 | 412 | 467 | 484 | 2.29 | 2.51 | 0.92 | 0.92 |

<Example 2>

Imaging of lipid droplets in cultured cells was studied by a comparative experiment on the performance between DBC30, DBC30-Bu, and DBC30-Ph, and a commercially available blue imaging reagent (Lipi-Blue). The investigated items were the luminescence intensity and the photostability.

Fig. 6 shows fluorescence imaging images obtained as follows. Each fluorescent reagent was added to a final concentration of 500 nM to HeLa cells cultured for 48 hours in the presence of 400 μM oleic acid. Culture was then performed for 60 minutes, and the cultured cells were irradiated with an excitation light (DBC30, DBC30-Bu, DBC30-Ph, Lipi-Blue: 430 nm) required for the imaging, followed by observation using a confocal laser microscope. It can be seen that the HeLa cells to which DBC30, DBC30-Bu, or DBC30-Ph was added exhibited higher fluorescence intensities than the HeLa cells to which Lipi-Blue was added.

Photostability is an important factor in long-term measurement for, for example, tracing the processes of formation, fusion, and degradation of lipid droplets. Each fluorescent reagent was added to a final concentration of 100 nM (DBC30, C6-OBu, or BODIPY493/503) or 2 μM (Lipi-Blue) to HeLa cells cultured for 48 hours in the presence of 400 μM oleic acid. Culture was then performed for 30 minutes, and the cultured cells were irradiated with an excitation light (DBC30, DBC30-Bu, Lipi-Blue: 430 nm; BODIPY 493/503: 488 nm) required for the imaging, followed by acquisition of imaging images using a confocal laser microscope. The photostability was evaluated by 500 times of scanning in which a set of 50 times of scanning was repeated. Fig. 7 shows plots of the fluorescence intensity ratio against the number of times of scanning. DBC30 was found to have higher photostability compared to the conventional reagents such as Lipi-Blue.

<Example 3>

In order to clarify the lipid droplet selectivities of DBC30, DBC30-Bu, and DBC30-Ph, co-staining experiments were carried out with BODIPY 493/503, which is a commercially available green fluorescent lipid droplet reagent (Figs. 8 to 10). Each fluorescent reagent was added to a final concentration of 100 nM to HeLa cells cultured for 48 hours in the presence of 400 μM oleic acid. Culture was then performed for 30 minutes, and the cultured cells were irradiated with an excitation light required for the imaging, followed by observation. For DBC30, DBC30-Bu and DBC30-Ph, the excitation wavelength was 430 nm, and the observation wavelength was 460-500 nm. For BODIPY 493/503, the excitation

wavelength was 488 nm, and the observation wavelength was 510 to 560 nm. Since the blue color (pseudocolor) of DBC30, DBC30-Bu, or DBC30-Ph and the green color (pseudocolor) of BODIPY 493/503 were found from the same sites in the cells, DBC30, DBC30-Bu, and DBC30-Ph were found to have lipid droplet selectivity.

<Example 4>

Since DBC30 shows fluorescence in the blue wavelength region, its use in co-staining with intracellular organelle-selective reagents showing green fluorescence or red fluorescence was studied. Mito Tracker Green (excitation wavelength, 488 nm; observation wavelength, 510 to 560 nm), which is a green fluorescent reagent showing accumulation in mitochondria, and Lyso Tracker Red (excitation wavelength, 570 nm; observation wavelength, >590 nm), which is a red fluorescent reagent showing accumulation in lysosomes, were used. As shown in Fig. 11, the fluorescent reagents showed distribution in different organelles in the cells (blue: lipid droplets; green: mitochondria; red: lysosomes), indicating that information on the shapes and sites of the organelles can be obtained. Thus, DBC30 can be used for multicolor imaging, and for combined use with a green fluorescent protein.

<Example 5>

Fluorescence imaging of lipid droplets in a tissue, and of an adipose tissue, using DBC30, DBC30-Bu, or DBC30-Ph is described below. Since the DBC30 series has remarkably low solubility in water (physiological saline), they cannot be easily directly dissolved. Furthermore, when a 5 mM stock solution of DBC30, DBC30-Bu, or DBC30-Ph in dimethyl sulfoxide (DMSO) is added (at a volume ratio of 10%) to physiological saline, precipitation of DBC30, DBC30-Bu, or DBC30-Ph occurs. Therefore, they cannot be administered to mice. In view of this, the 5 mM stock solution was added (at a volume ratio of 10%) to physiological saline supplemented with 10% bovine serum albumin. As a result, the precipitation of DBC30, DBC30-Bu, or DBC30-Ph could be suppressed. Regarding administration of MNs-NB, a stock solution in DMSO is directly administered to mice. Since administration of DMSO may cause death of mice from shock, the administration method in the present invention is thought to be a safer method. Here, 100 to 200 μL of 500 μM solution (50 to 100 nmol) was administered to the tail vein of mice under anesthesia, and a fluorescence imaging experiment was carried out. In the present experiment, a microscope capable of acquiring fluorescence lifetime images as well as fluorescence intensity images (FLIM: fluorescence lifetime imaging microscope) was used.

<Example 6>

Fatty-liver model mice are known to have a liver containing a larger amount of lipid droplets accumulated therein compared to normal mice. Here, fatty-liver model mice were prepared by feeding mice (BALB/cAJcl) with an ultra-high-fat, choline-deficient, methionine-reduced diet for 2 weeks. Fig. 12 shows fluorescence intensity imaging images and fluorescence lifetime imaging images (FLIM images) obtained by administration of 50 nmol of DBC30 to a normal mouse and a fatty-liver model mouse. In the mouse fed with a normal diet, small lipid droplets could be found in hepatocytes. On the other hand, in the fatty-liver model mouse (Week 2), large lipid droplets were imaged over the entire liver surface, thus indicating accumulation of lipid in the liver.

<Example 7>

In an individual, various adipose tissues and lipid droplets are present. Their imaging was carried out using DBC30. Fig. 13 shows fluorescence imaging images of a subcutaneous adipose tissue, an abdominal adipose tissue, skeletal muscle, and kidney, obtained by administration of DBC30 (50 nmol) to a mouse (BALB/cAJcl). Fluorescence derived from DBC30 was found from sites where lipid is accumulated. In particular, for the kidney, small lipid droplets distributed in tubular cells could be imaged. It can thus be expected that DBC30 may be useful as a tool for studying the association of lipid with lifestyle-related diseases such as diabetes, and with renal disfunction.

According to the above results, the compounds developed by the present invention including DBC30, DBC30-Bu, and DBC30-Ph are novel reagents that show blue fluorescence, and that are capable of imaging of intracellular lipid droplets, and adipose tissues and lipid droplets in living individuals.

INDUSTRIAL APPLICABILITY

The present invention can be used for fluorescence imaging of adipose tissues and lipid droplets in biological samples and living individuals.

## Claims

1. A lipid droplet detection reagent comprising a compound represented by the following General Formula (I):

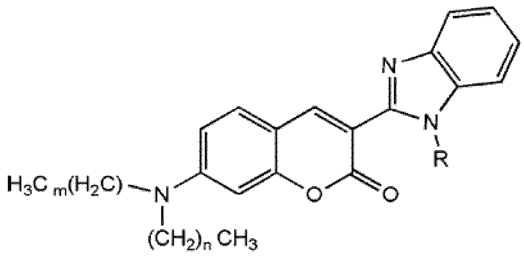

(I)

wherein

m represents an integer of 3 to 5;
n represents an integer of 3 to 5; and
R is $C_1$-$C_5$ alkyl, $C_6$-$C_{12}$ aryl, $C_7$-$C_{13}$ aralkyl, or hydrogen.

2. The detection reagent according to claim 1, wherein R is $C_1$-$C_4$ alkyl, or phenyl.

3. The detection reagent according to claim 1 or 2, wherein m and n are 3.

4. The detection reagent according to any one of claims 1 to 3, for the detection of a lipid droplet in a biological sample.

5. The detection reagent according to claim 4, wherein the biological sample is a cell or a tissue.

6. The detection reagent according to any one of claims 1 to 3, for the detection of a lipid droplet in a living individual.

7. A lipid droplet detection method comprising the step of:
administering the detection reagent according to any one of claims 1 to 6 to a biological sample or a living individual (other than a human).

8. A compound represented by the following General Formula (I)':

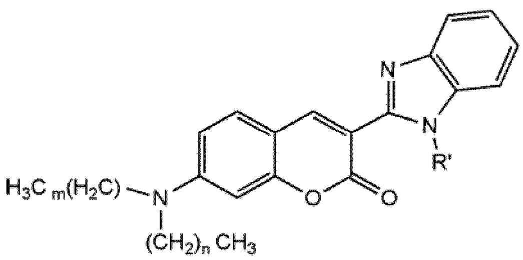

(I)'

wherein

m represents an integer of 3 to 5;
n represents an integer of 3 to 5; and
R' is $C_1$-$C_5$ alkyl, $C_6$-$C_{12}$ aryl, or $C_7$-$C_{13}$ aralkyl.

9. The compound according to claim 8, wherein R' is $C_1$-$C_4$ alkyl, or phenyl.

10. The compound according to claim 8 or 9, wherein m and n are 3.

Fig. 1

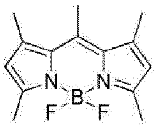

BODIPY493/503

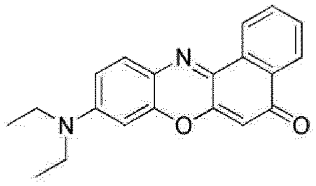

Nile Red

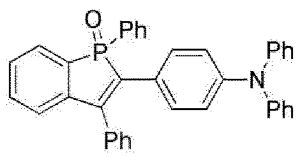

LipiDye

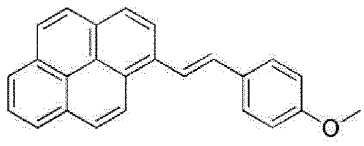

Lipi-Blue

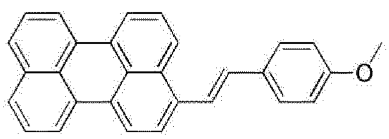

Lipi-Green

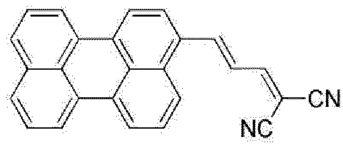

Lipi-Red

Fig. 2

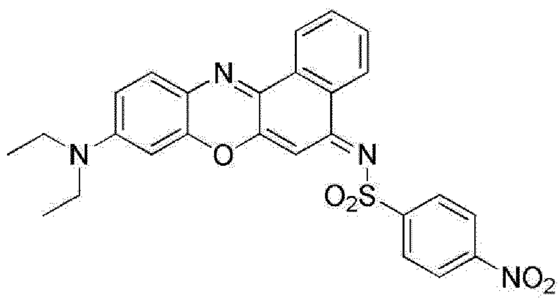

MNs-NB

Fig. 3

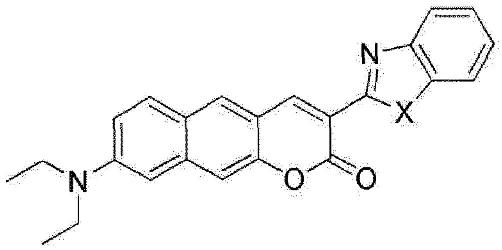

X : O (PC6O)
S (PC6S)
NH (PC6NH)
NMe (PC6NMe)

PC6X

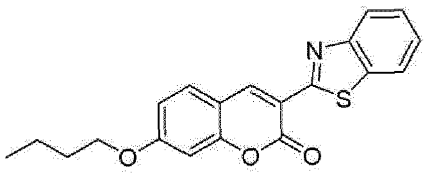

C6-OBu

Fig. 4

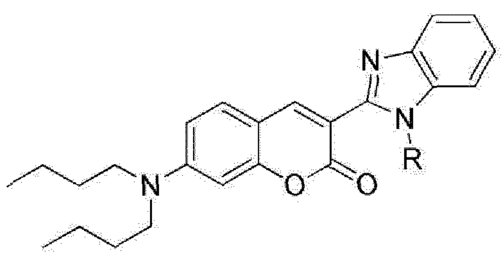

R : Me(DBC30)
Bu(DBC30-Bu)
Ph(DBC30-Ph)

Fig. 5

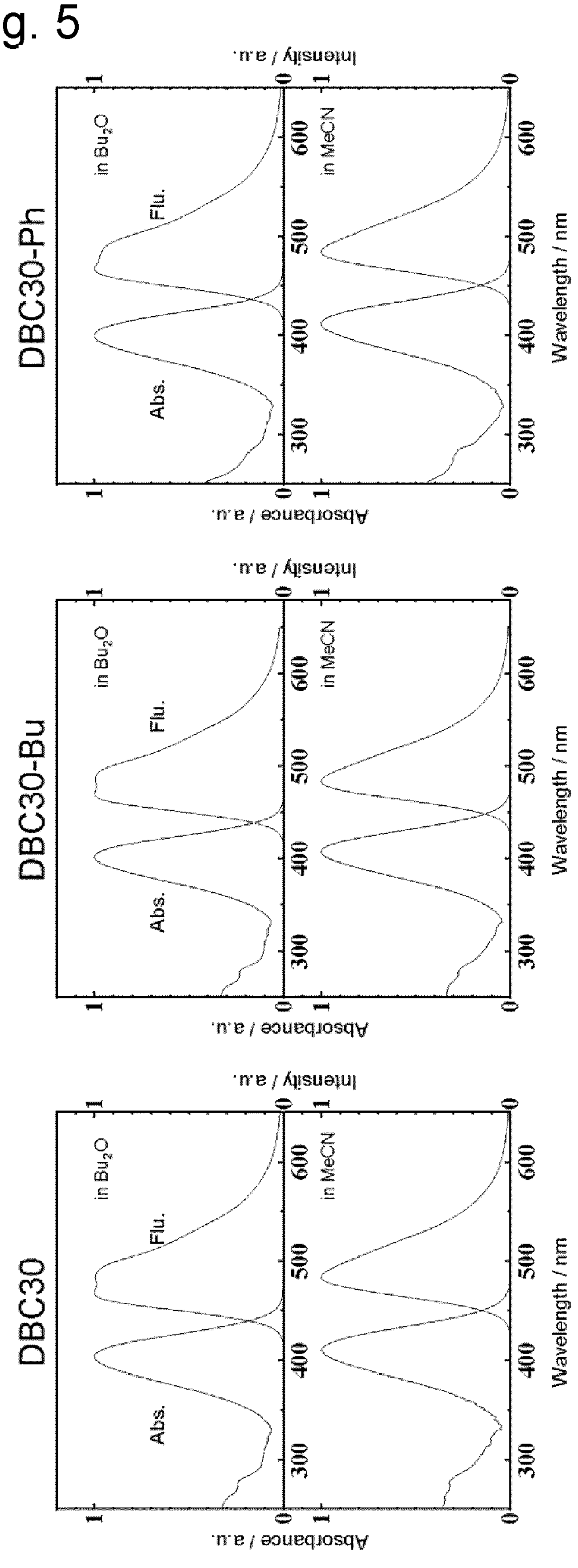
DBC30-Ph
DBC30-Bu
DBC30
Abs.
Flu.
in $Bu_2O$
in MeCN
Absorbance / a.u.
Intensity / a.u.
Wavelength / nm
300
400
500
600
0
1

Fig. 6

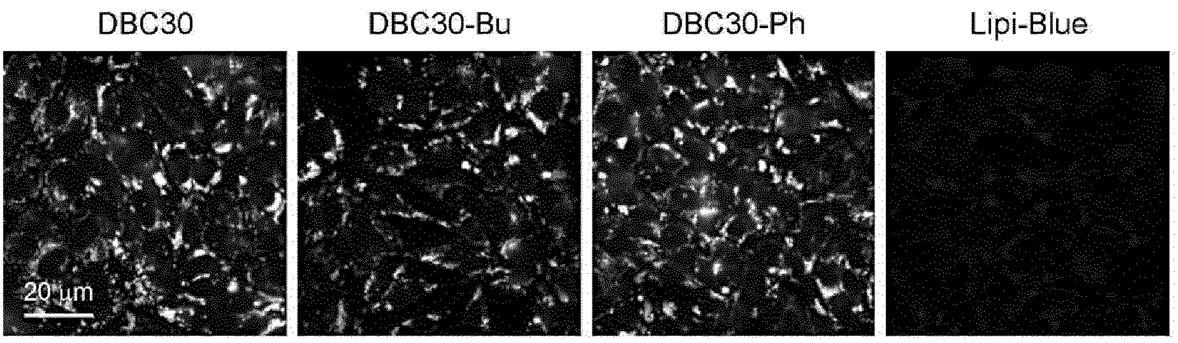

Fig. 7

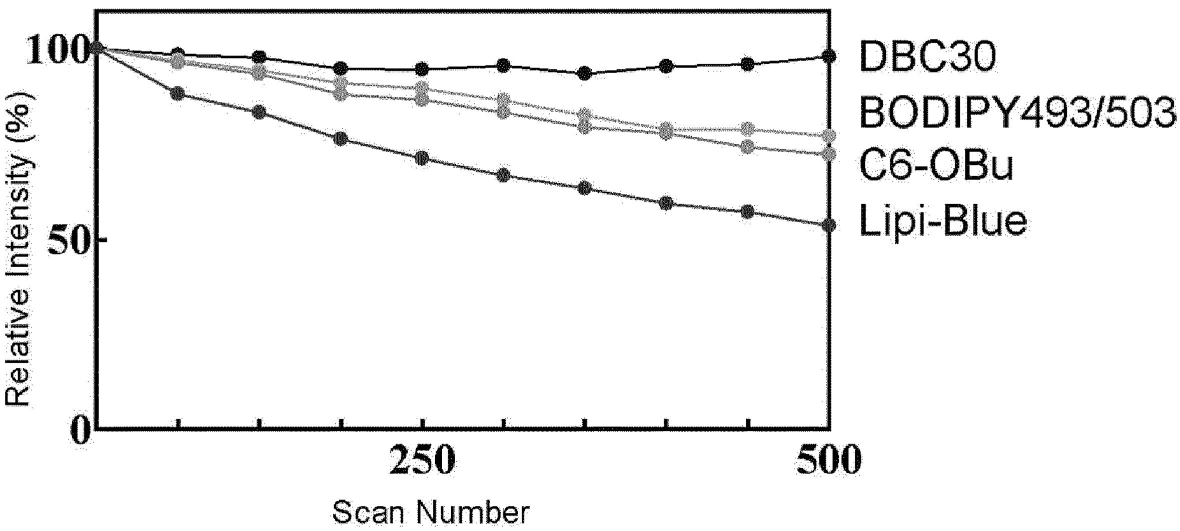
100
50
0
250
500
Relative Intensity (%)
Scan Number
DBC30
BODIPY493/503
C6-OBu
Lipi-Blue

Fig. 8

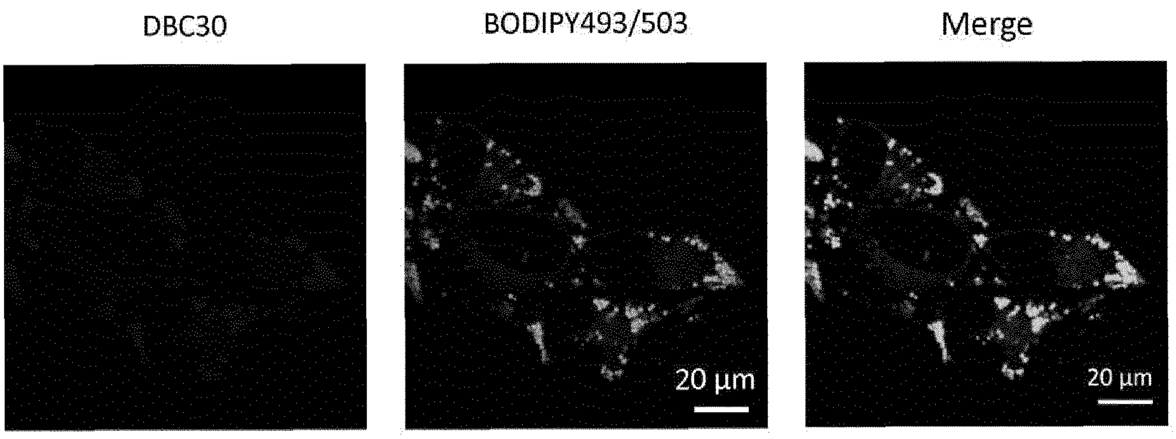

Fig. 9

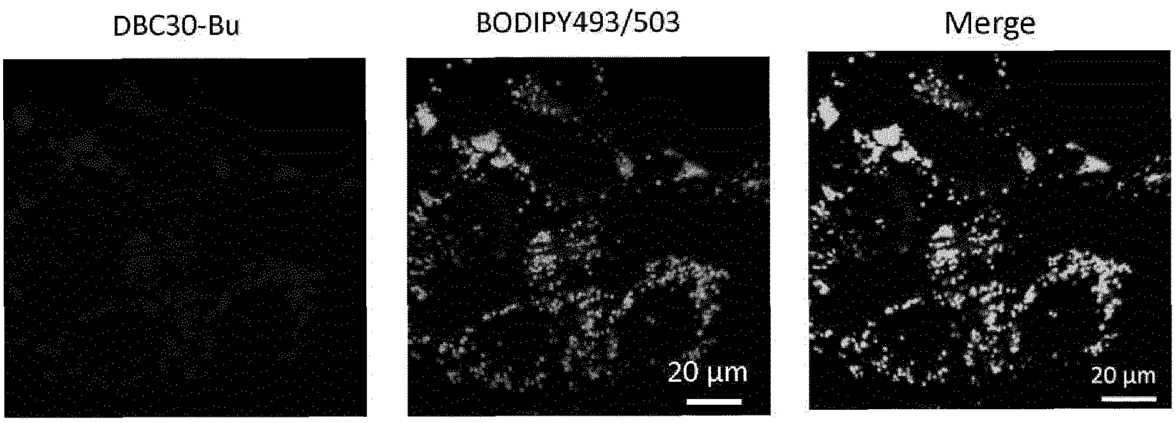

Fig. 10

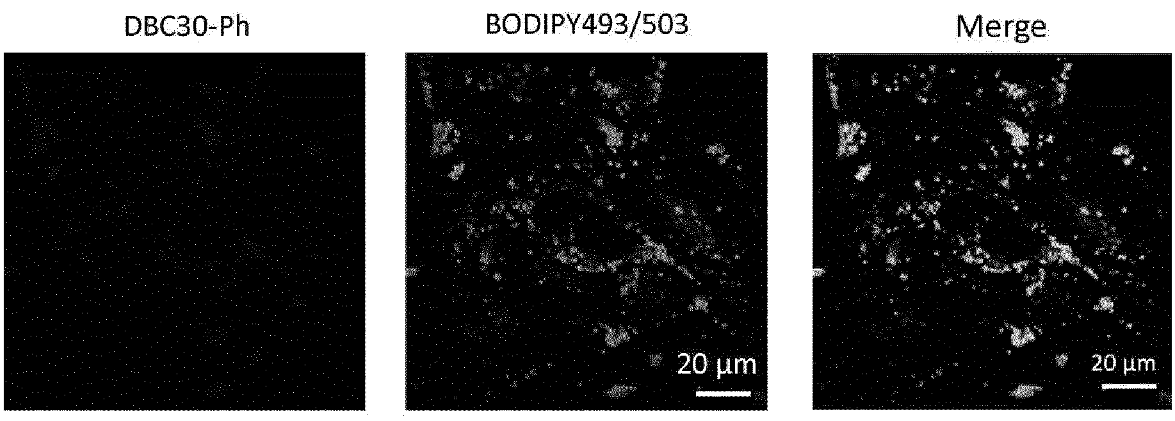

Fig. 11

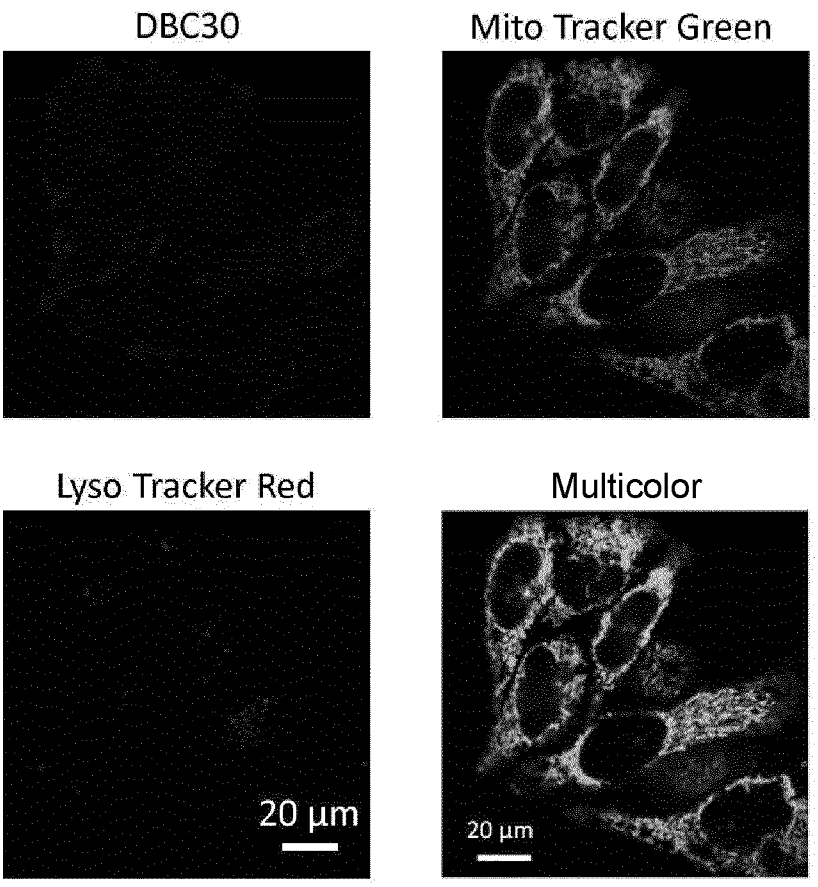
DBC30
Mito Tracker Green
Lyso Tracker Red
Multicolor
20 µm
20 µm

Fig. 12

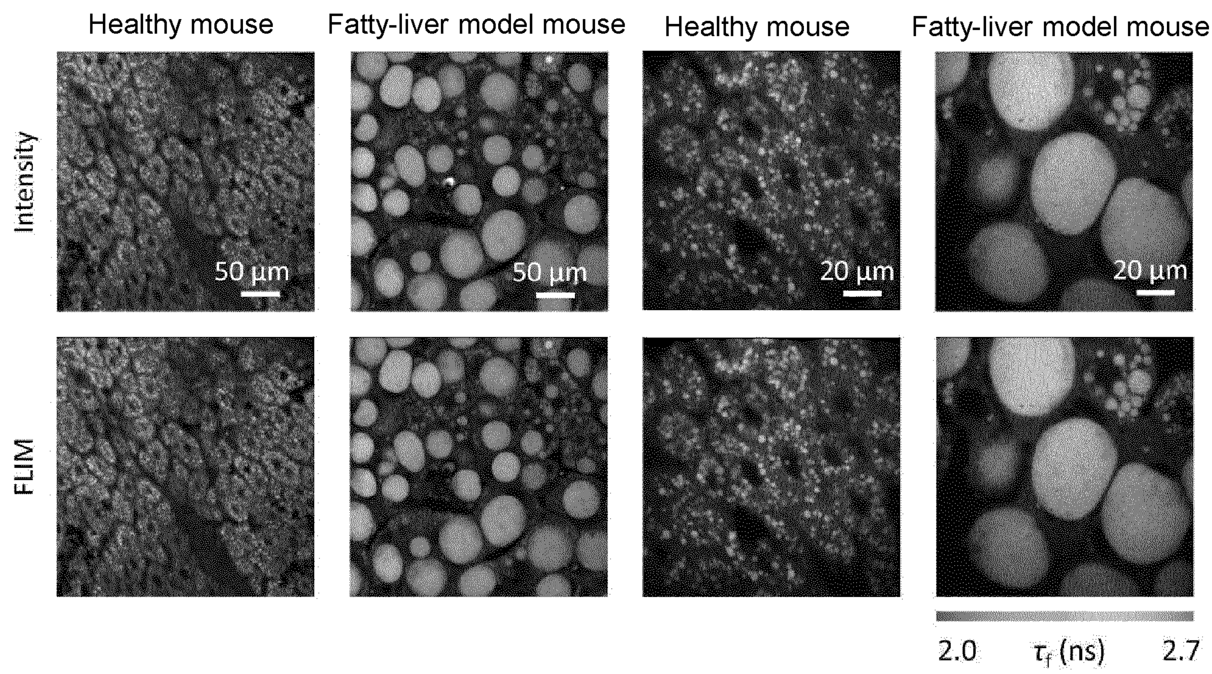
Healthy mouse
Fatty-liver model mouse
Healthy mouse
Fatty-liver model mouse
Intensity
FLIM
50 µm
50 µm
20 µm
20 µm
2.0
$\tau_f$ (ns)
2.7

Fig. 13

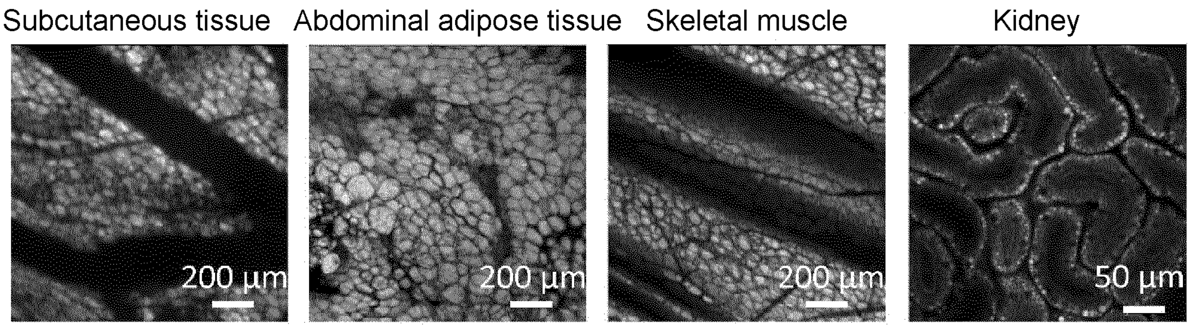
Subcutaneous tissue
Abdominal adipose tissue
Skeletal muscle
Kidney
200 µm
200 µm
200 µm
50 µm

**INTERNATIONAL SEARCH REPORT**

International application No.
**PCT/JP2022/040063**

**A. CLASSIFICATION OF SUBJECT MATTER**

***G01N 21/78***(2006.01)i; ***A61K 49/00***(2006.01)i; ***C07D 405/04***(2006.01)i; ***C09B 57/02***(2006.01)i; ***G01N 33/50***(2006.01)i; ***G01N 33/92***(2006.01)i
FI: G01N21/78 C; A61K49/00; C07D405/04; C09B57/02 D CSP; G01N33/50 Z; G01N33/92 A; G01N33/92 C

According to International Patent Classification (IPC) or to both national classification and IPC

**B. FIELDS SEARCHED**

Minimum documentation searched (classification system followed by classification symbols)

G01N21/78; A61K49/00; C07D405/04; C09B57/02; G01N33/50; G01N33/92

Documentation searched other than minimum documentation to the extent that such documents are included in the fields searched

Published examined utility model applications of Japan 1922-1996
Published unexamined utility model applications of Japan 1971-2022
Registered utility model specifications of Japan 1996-2022
Published registered utility model applications of Japan 1994-2022

Electronic data base consulted during the international search (name of data base and, where practicable, search terms used)

JSTPlus/JMEDPlus/JST7580 (JDreamIII); CAplus/REGISTRY (STN)

**C. DOCUMENTS CONSIDERED TO BE RELEVANT**

| Category* | Citation of document, with indication, where appropriate, of the relevant passages | Relevant to claim No. |
|---|---|---|
| A | WO 2012/078308 A1 (HALUSHKA et al.) 14 June 2012 (2012-06-14) claim 11 | 1-10 |
| A | WO 2014/142320 A1 (RIKEN) 18 September 2014 (2014-09-18) | 1-10 |
| A | WO 2020/189721 A1 (NATIONAL UNIVERSITY CORPORATION GUNMA UNIVERSITY) 24 September 2020 (2020-09-24) | 1-10 |

☐ Further documents are listed in the continuation of Box C.　☑ See patent family annex.

* Special categories of cited documents:
"A" document defining the general state of the art which is not considered to be of particular relevance
"E" earlier application or patent but published on or after the international filing date
"L" document which may throw doubts on priority claim(s) or which is cited to establish the publication date of another citation or other special reason (as specified)
"O" document referring to an oral disclosure, use, exhibition or other means
"P" document published prior to the international filing date but later than the priority date claimed
"T" later document published after the international filing date or priority date and not in conflict with the application but cited to understand the principle or theory underlying the invention
"X" document of particular relevance; the claimed invention cannot be considered novel or cannot be considered to involve an inventive step when the document is taken alone
"Y" document of particular relevance; the claimed invention cannot be considered to involve an inventive step when the document is combined with one or more other such documents, such combination being obvious to a person skilled in the art
"&" document member of the same patent family

| Date of the actual completion of the international search | Date of mailing of the international search report |
|---|---|
| **22 December 2022** | **10 January 2023** |

| Name and mailing address of the ISA/JP | Authorized officer |
|---|---|
| **Japan Patent Office (ISA/JP)**<br>**3-4-3 Kasumigaseki, Chiyoda-ku, Tokyo 100-8915**<br>**Japan** | Telephone No. |

Form PCT/ISA/210 (second sheet) (January 2015)

**INTERNATIONAL SEARCH REPORT**
**Information on patent family members**

International application No.
**PCT/JP2022/040063**

| Patent document cited in search report | Publication date (day/month/year) | Patent family member(s) | Publication date (day/month/year) |
|---|---|---|---|
| WO 2012/078308 A1 | 14 June 2012 | US 2013/0344588 A1<br>claim 11 | |
| WO 2014/142320 A1 | 18 September 2014 | (Family: none) | |
| WO 2020/189721 A1 | 24 September 2020 | US 2022/0163545 A1<br>EP 3943919 A1<br>CN 113597547 A | |

Form PCT/ISA/210 (patent family annex) (January 2015)

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- JP 6241014 B
- WO 2020189721 A